# EUROPEAN PATENT SPECIFICATION

(11) **EP 2 217 189 B1**
(45) Date of publication and mention of the grant of the patent: **18.01.2012**
(21) Application number: 08857125.2
(22) Date of filing: 05.12.2008
(51) Int. Cl.: A61F 13/00

(54) **IMPROVED COMPRESSION BANDAGE STRUCTURES**
VERBESSERTE KOMPRESSIONSVERBANDSTRUKTUREN
STRUCTURES AMÉLIORÉES DE BANDAGE DE COMPRESSION

(30) Priority: 07.12.2007 GB 0724028
(43) Date of publication of application: 18.08.2010
(73) Proprietor: Medlock Medical Limited, Oldham OL1 3HS (GB)
(72) Inventor: LEEMING, Ray, Shaw, Oldham OL2 7AR (GB); WOOSEY, Karen, Rachel, Altrincham, Cheshire WA14 5UY (GB)
(74) Representative: Wilson Gunn
(86) International application number: PCT/GB2008/004009
(87) International publication number: WO 2009/071894

(56) References cited:
- EP-A- 1 052 319
- WO-A-95/16416
- DE-A1- 10 260 694
- DE-U1- 29 506 824
- US-A- 5 807 295
- US-A1- 2001 007 929
- US-A1- 2005 159 066
- US-A1- 2006 229 544
- US-A1- 2006 253 058
- US-A1- 2008 082 035
- US-B1- 6 918 140

## Description

### TECHNICAL FIELD

This invention relates to improved compression bandage structures.

### BACKGROUND ART

Compression bandages are used to facilitate healing. For example, a compression bandage wound around a patient's leg to give a pressure gradient up the leg can facilitate the healing of venous ulcers.

Knitted and woven stretchable fabrics are used for this purpose.
However, with known fabrics the pressure applied in use can cause discomfort to the patient at pressure points, e.g. around bony prominences, and may give rise to chafing or injury. To avoid this it may be necessary to introduce padding beneath the bandage.

Softer bandage fabrics are known such as non-woven or felted fabrics, but these do not have sufficient strength in themselves to permit application of satisfactory levels of compression. They are commonly used for comfort as padding beneath plaster casts and compression bandages.

Current bandaging methods used in the treatment of venous leg ulcers use two or more, possibly up to four, individual bandages applied in layers to build up to the correct sub-layer pressures. Usually, the last (outermost) applied layer is a cohesive bandage which secures the sub-layers and helps to mould the dressing to the shape of the limb.

A four-layer bandage was developed during the 1980's at Charing Cross Hospital, London and trailed on the Vascular department to improve the care and treatment of patients with venous ulceration. Single elasticated bandages were being used at that time but presented difficulties in achieving the required levels of sub-layer pressures appropriate to encourage the healing process, and in maintaining that pressure over a period of time. The four layer bandage method was designed to apply a high level of compression (35-40mm Hg) using a simple crêpe bandage, a light compression bandage and a soft padding bandage to build up in layers to give the cumulative pressure required, and an outer cohesive bandage to secure and hold the lower layers in place, for at least a week.

WO 95/16416 disclosed a compression bandage which is characterised by the use of a spacer fabric having opposite face structures united and held in spaced disposition by threads.

By using a spacer fabric it was possible to attain high levels of compression with a soft, comfortable padding structure.

### SUMMARY OF THE INVENTION

An object of the present invention is to provide a compression bandage of this type which is improved to provide a more cohesive bandage.

The present invention provides a multi-layered bandage as defined in claim 1, comprising, an inner wound facing layer; an intermediate layer including a spacer fabric having opposite face structures united and held in spaced disposition by threads; and an outer layer comprising a mixture of non-elastic yarns and elasticated threads.

The outer layer is covered on its exterior surface with a cohesive coating. This may be applied after manufacture of the bandage, and the coating comprises a latex or non-latex adhesive.

The non-elastics yarns of the outer layer may comprise viscose or cotton yarns.

The inner wound facing layer preferably incorporates absorbent viscose fibres, and may further include bacteriostatic yarns including silver-based bactericide, and/or polysaccharide fibres, and/or a proportion of elastic yarns or fibres to provide a degree of compression.

The intermediate layer incorporates auxetic materials to permit maintenance of a steady consistent pressure.

The application of adhesive to form the cohesive coating may be carried out in-line, but following steam sterilisation.

The inner, intermediate, and outer layers may be made by a knitting operation, particularly warp knitting; or alternatively it can be weft knitted to form a tubular spacer fabric, or produced on weaving machinery to produce a woven spacer fabric.

The intermediate layer may comprise a spacer fabric formed on a double needle bar plush machine. It is already known to form such fabrics but hitherto the fabric has been separated between the face structures by severing the spacer threads to give two plus-faced fabrics, or the spacer fabric has been used without separation as a structural or insulting lining or cover. The present invention is based in the realisation that spacer fabrics can be used to advantage in the field of compression bandages.

Any suitable materials or combination of materials may be used for the spacer fabric.

Preferably the outer face structure i.e. that which in use faces away from the patient's skin, is formed from multifilament yarns such as nylon or polyester alone or in combination with durable, elastic yarns, particularly multifilaments or monofilaments for example of polyurethane fibres such as Elastane or that sold under the registered trade mark Lycra.

Preferably the inner face structure, i.e. that which faces the patient's skin in use, is formed from a suitably comfortable, or skin compatible material, particularly cotton and/or viscose yarns alone or in combination with elastic yarns.

It is also possible to have cotton or other absorbent yarns away from the patient's skin so as to encourage flow of moisture away from the body surface.

The spacing threads are preferably relatively strong, self-supporting threads, which, suitably may be monofilaments or multifilaments of a polyester or nylon or other suitable materials, such as Coolmax which is a four channel Dacron polyester fibre with a special cross-section structure, selected to give the desired capillary action and resilience or padding effect.

Preferably also, the threads run between the face structures in two or more sets respectively at two or more opposite inclinations, thereby to provide resistance to lateral displacement i.e. to displacement of the face structures in opposite directions parallel to such structures.

The fabric may have markers provided thereon to assist in application of predetermined levels of compression. The markers which are preferably geometric may be arranged so that they distort visually on stretching whereby the required level of compression is attained when the distortion is of a predetermined nature. For example, the marks may comprise printed rectangles which are stretched to become squares, or ovals which become circles, or other shapes.

There may be two or more sets of markers corresponding to two or more levels of compression.

The bandage of the invention may have any suitable degree and configuration of elasticity. Most preferably it has a elongation in the range 30% to 120% in a longitudinal direction of the bandage. It is however also possible to have elasticity in the transverse direction, and also the elongation may be outside the above range perhaps as low as 10% or as high as 200%.

The padding characteristics of the bandage will depend on the spacing in the intermediate layers between the face structures and the characteristics of the spacer threads. Spacings in the range 1.5mm to 60mm are possible depending on requirements, (a preferred range being 2.0 to 5.00mm), and the spacer threads may have a linear density of 8 to 300 decitex, particularly 10 to 100 decitex and preferably 1.5-85 decitex. Multifilament or monofilament synthetic yarns used in the face structures may have linear densities in the range 22-30decitex. Where used cotton yam may be single or folded having a count Ne 1 /30 to 1 /60 or 2/60 to 2/1 20.

The spacer fabric may be finished in the usual way with a stentor and application of heat/steam to give desired properties, and, if desired the fabric may be surface finished e.g. by brushing.

The fabric may be cut to give bandages of varying lengths, usually 6 meters.

The invention also provides a method of forming a compression binding in situ wherein a bandage as described above is wrapped around an area of a patient's body such as limbs with varicose ulcers and maintained in position in compression thereon. The compression may be in the range 10-50mm Hg.

The bandage may be used alone or with other materials or fabrics. Thus, in some cases it may be desirable to introduce padding separate to the bandage, and/or to apply additionally conventional compression and/or crêpe bandages.

An embodiment of compression bandage structure in accordance with the invention will now be described by way of example with reference to the accompanying drawings, wherein:-
- Figure 1: is a diagrammatic sectional view of a multi-layer bandage structure according to the invention;
- Figure 2: is a diagrammatic, and partially sectional view of a sample of the bandage structure of Figure 1; and
- Figure 3: is a diagrammatic perspective view of the knitting pattern of an intermediate layer forming part of the bandage structure.

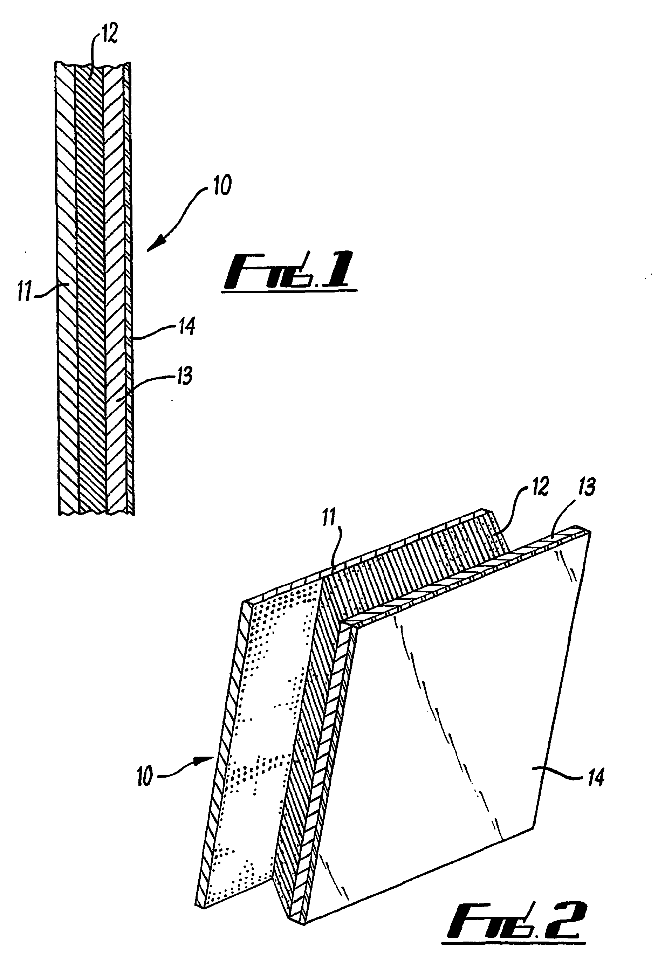

An enlarged section of a fragment of a bandage structure 10 in accordance with the invention is shown in Figure 1. The structure 10 comprises four layers, namely an inner facing layer 11 which contacts the patient's skin and overlies the wound. This is a knit of viscose fibre yarns, which includes bacteriostatic yarns which contain a silver based antibacterial preparation, such as SKINLIFE (Trade Mark), and also polysaccharide fibres, and a proportion of elastic yarns or fibres to provide a degree of compression.

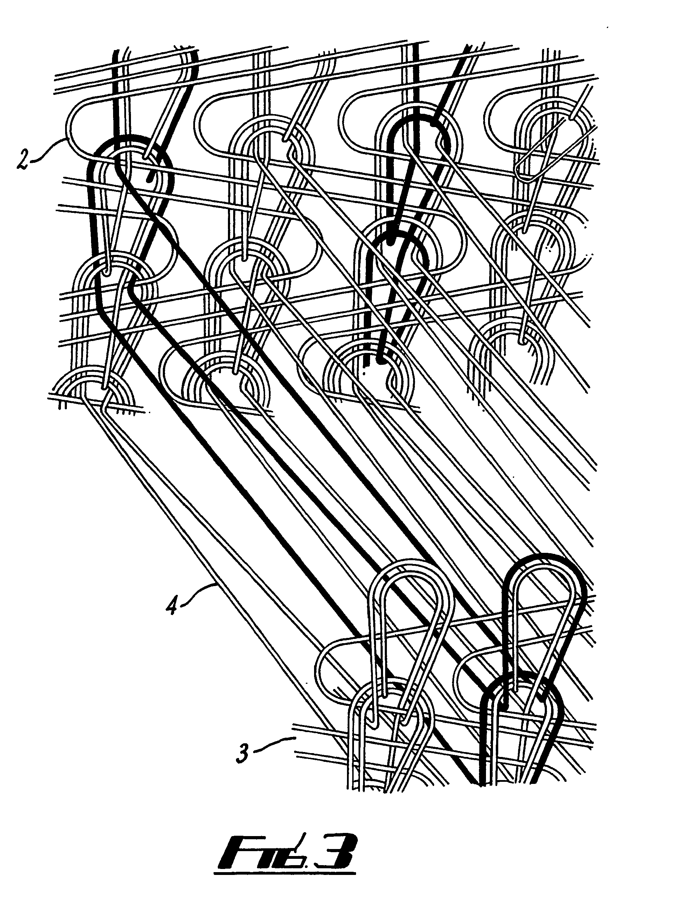

The second layer of the structure comprises an intermediate layer 12 which comprises a spacing layer, of about 3mm thickness. This provides a soft padding layer, with an ability to retain wound exudates. This additionally allows a degree of "breathability" to the dressing by incorporating yarns such as COOLMAX (Trade Mark). This layer 12 incorporates auxetic materials which permit a steady and consistent pressure profile to be maintained. The structure of this intermediate layer 12 is substantially similar to the bandage structure described in WO 95/16416 and a small part of this is shown in Figure 3 of the drawings of this application.

The outer face structure 2 is formed from nylon multifilaments (as the pillar stitches) and elastic multifilaments or monofilaments e.g. Lycra (registered trade mark) (for the linking face stitches).

The inner face structure 3 is formed from cotton yarns (as the pillar stitches), and elastic multifilaments e.g. Lycra (for the linking face stitches).

The spacer threads 4 are polyester or nylon monofilaments. Fig. 1 shows spacer threads 4 which are all parallel to each other for the sake of clarity. There are two sets of spacer threads 4, one set as shown and the other set crossing these with the same inclination in the opposite direction.

The spacer threads 4 act to hold the face structures 2, 3 apart in relatively fixed relationship with regard to directions parallel and perpendicular to the faces. Compression of the fabric is resisted by the threads whereby the fabric has the nature of a resiliently compressible padding material.

The bandage 1 is also stretchable longitudinally as a consequence of the knit structure, particularly the stitches, and the elastic yarns used.

The spacer fabric comprising layer 12 is finished with a stentor to give desired elongation which would typically be in the range of 30-120% in the longitudinal direction.

Returning to Figures 1 and 2, the outer face of the intermediate layer 12 is covered by an outer layer 13 which comprises a mixture of viscose and/or cotton yarns, which are substantially inelastic, with elasticated threads, e.g. of Elastane which provide additional compressive strength for compression.

Finally, the bandage structure includes an outer cohesive coating 14 which is applied to the exterior of the outer layer 13 after knitting of the bandage structure, by a process which includes a steam sterilisation step, and application of a latex or non latex adhesive.

The resulting multi-layer bandage structure 10 is configured to provide a firm but gentle pressure over the wound, and to promote healing and anti-sepsis. Use of the bandage 10 will enable the practice of using a plurality of wound bandages with a single multi layered compression bandage.

## Claims

1. A multi-layered bandage comprising a spacer fabric (12) forming an intermediate layer having opposite face structures united and held in spaced disposition by threads, **characterised in that** the bandage further comprises an inner wound-facing layer (11) and an outer layer (13) comprising a mixture of non-elastic yarns and elasticated threads, and **in that** the outer layer (13) is covered on an exterior surface with a cohesive coat (14), which comprises a latex or non-latex adhesive, and **in that** the intermediate layer incorporates auxetic materials to permit maintenance of a steady consistent pressure.

2. A bandage according to claim 1, **characterised in that** the non-elastic yarns of the outer layer (13) comprise viscose or cotton yarns.

3. A bandage according to claim 1 or claim 2, **characterised in that** the inner wound-facing layer (11) incorporates absorbent viscose fibres.

4. A bandage according to claim 3 **characterised in that** the inner wound-facing layer further includes bacteriostatic yarns including a silver-based bactericide and/or polysaccharide fibres and/or a proportion of elastic yarns or fibres.

5. A bandage according to claim 1, wherein the cohesive coating (14) is applied as an adhesive in-line, following a steam sterilisation step.

## Patentansprüche

1. Mehrfachschichtverband mit einem Füllgewebe (12), das eine Zwischenschicht bildet, mit gegenüberliegenden Strukturen, die durch Fäden verbunden und in beabstandeter Anordnung gehalten sind,
**dadurch gekennzeichnet,**
**dass** der Verband ferner eine innere wundenaufliegende Schicht (11) und eine äußere Schicht (13) mit einer Mischung aus nicht-elastischen Garnen und elastifizierten Fäden aufweist, und dass die Außenschicht (13) auf einer Außenoberfläche mit einer kohäsiven Beschichtung (14) bedeckt ist, die einen Latex oder Nicht-Latex-Klebstoff enthält, und dass die Zwischenschicht auxetische Materialien enthält, um die Aufrechterhaltung eines gleichbleibenden stetigen Druckes zu ermöglichen.

2. Verband nach Anspruch 1,
**dadurch gekennzeichnet,**
**dass** die nicht-elastischen Garne der Außenschicht (13) Viskose- oder Baumwollgarne aufweisen.

3. Verband nach Anspruch 1 oder Anspruch 2,
**dadurch gekennzeichnet,**
**dass** die innere wundenaufliegende Schicht (11) absorbierende Viskosefasern enthält.

4. Verband nach Anspruch 3,
**dadurch gekennzeichnet,**
**dass** die innere wundenaufliegende Schicht ferner bakteriostatische Garne einschließlich einem auf Silber basierendem Bakterizid und/oder Polysaccharidfasern und/oder einen Anteil an elastischen Garnen oder Fasern enthält.

5. Verband nach Anspruch 1,
wobei die kohäsive Beschichtung (14) als ein Zwischenklebstoff, nachfolgend einem Dampfstabilisierungsschritt, aufgetragen ist.

## Revendications

1. Bandage multicouche comprenant une étoffe d'écartement (12) formant une couche intermédiaire ayant des structures de faces opposées solidaires et maintenues en disposition espacée par des fils, **caractérisé en ce que** le bandage comprend en outre une couche intérieure tournée vers la plaie (11) et une couche extérieure (13) comprenant un mélange de brins non élastiques et de fils élastiques, et **en ce que** la couche extérieure (13) est recouverte sur une surface extérieure par un revêtement cohésif (14), qui comprend un adhésif à base de latex ou sans latex, et **en ce que** la couche intermédiaire contient des matériaux auxétiques pour permettre le maintien d'une pression constante stable.

2. Bandage selon la revendication 1, **caractérisé en ce que** les brins non élastiques de la couche extérieure (13) comprennent des brins de viscose ou de coton.

3. Bandage selon la revendication 1 ou la revendication 2, **caractérisé en ce que** la couche tournée vers la plaie (11) contient des fibres de viscose absorbantes.

4. Bandage selon la revendication 3, **caractérisé en ce que** la couche tournée vers la plaie contient en outre des brins bactériostatiques contenant un bactéricide à base d'argent et/ou des fibres de polysaccharide et/ou une certaine proportion de brins ou fibres élastiques.

5. Bandage selon la revendication 1, dans lequel le revêtement cohésif (14) est appliqué sous la forme d'un adhésif en ligne après une étape de stabilisation à la vapeur.
